# FASCICULE DE BREVET EUROPEEN

(11) **EP 1 476 433 B1**
(45) Date de publication et mention de la délivrance du brevet: **09.11.2005**
(21) Numéro de dépôt: 03739537.3
(22) Date de dépôt: 14.02.2003
(51) Int. Cl.: C07D 233/06, C07D 233/20, C07D 233/22, C07C 69/00, A61K 31/4164, A61P 25/00

(54) **NOUVEAUX DERIVES DE TRICYCLO-IMIDAZOLINES, LEUR PROCEDE DE PREPARATION ET LEUR UTILISATION A TITRE DE MEDICAMENTS**
TRIZYKLO-IMIDAZOLINEN DERIVATEN, VERFAHREN ZU DEREN HERSTELLUNG UND DEREN VERWENDUNG ALS HEILMITTELN
NOVEL TRICYCLOIMIDAZOLINE DERIVATIVES, METHOD FOR PRODUCTION AND USE THEREOF AS MEDICAMENTS

(30) Priorité: 14.02.2002 FR 0201839
(43) Date de publication de la demande: 17.11.2004
(73) Titulaire: PIERRE FABRE MEDICAMENT, 92100 Boulogne (FR)
(72) Inventeur: VACHER, Bernard, F-81100 Castres (FR); BONNAUD, Bernard, F-81090 Lagarrigue (FR); MARIEN, Marc, F-81100 Castres (FR); IMBERT, Thierry, F-81290 Viviers-les-Montagnes (FR)
(74) Mandataire: Warcoin, Jacques
(86) Numéro de dépôt international: PCT/FR2003/000480
(87) Numéro de publication internationale: WO 2003/068755

(56) Documents cités:
- WO-A-01/85698
- C.F. KOELSCH: "AN INDONE TO NAPHTOL RING EXPANSION." JOURNAL OF ORGANIC CHEMISTRY., vol. 26, 1961, pages 1003-5, XP002245844 AMERICAN CHEMICAL SOCIETY. EASTON., US ISSN: 0022-3263

## Description

La présente invention concerne de nouveaux dérivés tricycliques substitués par un groupe 4,5-dihydro-1H-imidazole. Les composés de l'invention interagissent sélectivement avec les récepteurs adrénergique du type alpha-2 pré- et/ou post-synaptiques (J. Neurochem. 2001, 78, 685-93) au niveau desquels ils se comportent comme des agonistes partiels, des antagonistes ou des agonistes inverses. En tant que tel, les composés de l'invention sont donc potentiellement utiles dans le traitement des pathologies ou des conditions sensibles à une régulation adrénergique contrôlée par les récepteurs alpha-2 adrénergiques. La liste des pathologies considérées comme sensibles à une telle régulation est excessivement longue. Le champ d'application de la présente invention se limite, toutefois, au traitement des maladies neurodégénératives ainsi qu'au traitement de l'évolution de celles-ci (Psychopharmacology 1996, 123(3), 239-49; Prog. Neuro-Psychopharmacol. Biol. Psychiatry 1999, 23(7), 1237-46; FR 2789681 ; WO 9835670 ; WO 9806393 ; WO 9500145 ; WO 9413285), en particulier au traitement de la maladie ou au traitement de l'évolution de la maladie d'Alzheimer (US 5281607; FR 2795727; WO 9501791; WO 9415603).

La maladie d'Alzheimer est la maladie dégénérative progressive la plus répandue dans la population âgée. Il est estimé que plus de 15 millions de personnes en sont atteintes (New Engl. J. Med. 1999, 341(22), 1670-79 ; Drug Benefit Trends 2001, 13/7, 27-40). A l'heure actuelle, les inhibiteurs d'acétylcholinestérase (e.g., tacrine, donepezil, rivastigmine et galantamine) constituent la stratégie thérapeutique principale. Cette approche thérapeutique est, cependant, purement symptomatique et les bénéfices thérapeutiques obtenues sont, tout au plus, modestes (Drugs 2001, 61/1, 41-52). Les options thérapeutiques efficaces contre la maladie d'Alzheimer étant limitées (Curr. Opin. Invest. Drugs 2001, 2(5), 654-56), la découverte de nouveaux traitements mettant en oeuvre des molécules dotées d'un mécanisme d'action différent de celui des molécules actuellement disponibles en clinique et capables de traiter ou de retarder l'évolution de la maladie est donc fortement souhaitable.

Il a été montré, in vitro et chez l'animal, qu'une substance activant le système noradrénergique peut s'opposer à la progression de la dégénérescence des neurones (J. Neurophysiol. 1998, 79(6), 2941-63 ; Pharmacol. Biochem. Behav. 1997, 56(4), 649-55 ; J. Cereb. Blood Flow Metabolism 1990, 10(6), 885-94) et, de plus, a la propriété de stimuler la croissance neuronale (J. Comp. Neurol. 1974, 155(1), 15-42 ; Neuroscience 1979, 4(11), 1569-82 ; Neuroreport 1991, 2, 528-8). Il s'ensuit que des composés possédant des propriétés antagonistes ou agonistes inverses au niveau des récepteurs alpha-2 adrénergique, en particulier au niveau des récepteurs alpha-2 pré-synaptiques, peuvent être utiles dans le traitement des maladies neurodégénératives. Compte tenu du potentiel thérapeutique des composés dotés d'une activité antagoniste ou agoniste inverse pour les récepteur alpha-2 adrénergiques la découverte de structures nouvelles dotés de telles propriétés est fortement souhaitable. A ce titre, la demanderesse a découvert que des dérivés tricycliques substitués par un groupe 4,5-dihydro-1H-imidazole interagissent sélectivement avec les récepteurs adrénergique du sous type alpha-2 au niveau desquels ils se comportent comme des antagonistes ou des agonistes inverses.

De nombreux antagonistes et/ou antagonistes partiels pré- et/ou post-synaptiques des récepteurs alpha-2 adrénergique sont connus et décrits dans la littérature. Bien que les composés en question appartiennent à des classes chimiques différentes (Idrugs 2001, 4(6), 662-76), certains comportent dans leur structure chimique un motif commun du type 4,5-dihydro-1H-imidazole. Parmi ces derniers, on peut citer, à titre d'exemples, des composés du type :
. dihydro-isoindole-méthyl- (EP 275639 ; EP 313288 ; EP 413433 ; US 4959374 ; WO 9309113 et Eur. J. Pharmacol. 1989, 168(3), 381-6);
. 2,3-dihydro-indole- (FR 2577223 ; US 4908376 et US 4912125) ;
. 5,6-dihydro-thieno[3,4-c]pyrrol-méthyl- (EP 682028) ;
. 1,2,4,5-tetrahydro-1H-pyrrolo[3,2,1]indole- (FR 2583048; FR 2611717 ; DE 4325491 et Life Sci. 1998, 62(9), 839-52) ;
. 1,2,3,4-tetrahydro-isoquinoline-methyl- (J. Med. Chem. 1990, 33(2), 596-600) ;
. 1,2,3,4-tetrahydro-quinoline- (US 5017584 ; JP 60058976 et FR 2576308);
. 2,3-dihydro-benzochromene- (Heterocycles 2001, 55(2), 387-92) ;
. 1,4-benzodioxane- (EP 33655 ; EP 58006 ; EP 92328 ; Neurochem. Int. 1996, 30(1), 47-53 et Mol. Neuropharmacol. 1992, 1(4), 219-24) ;
. 1,4-benzodioxane-methyl- (EP 74711) ;
. 4H-1,3-benzodioxine- (J. Pharmacol. Exp. Ther. 1995, 272(2), 681-8) ;
. 2,3-dihydro-1,4-benzoxathiine- (Eur. J. Med. Chem. 1987, 22(4), 273-6) ;
. 2H-1-benzopyrane- et naphtalène- et 3,4-dihydro-naphtalene-(Bioorg. Med. Chem. 1997, 5(5), 843-56) ;
. dibenzo[1,4]dioxépine- et dibenzopyrane- (Eur. J. Med. Chem. 1991, 26(2), 207-13) ;
. 2,3-dihydro-2-benzofurane- (US 4411908 et WO 9205171) ;
. furopyridine- analogues de l'éfaroxan (Heterocycles 1998, 48(12), 2529-34) ;
. 2-benzofurane- (Eur. J. Pharmacol. 1996, 304(1-3), 221-29 et Eur. J. Pharmacol. 1998, 353(1), 123-35) ;
. 1-hydroxy-2-phenoxy-2-phenyl-ethyl- (Eur. J. Med. Chem. 1990, 25(9), 757-63 ;
. phénoxy-phényl-éthyl- (EP 423802) ;
. des analogues de la cirazoline (Bioorg. Med. Chem. 2000, 8(5), 883-88) ;
. des analogues de la phentolamine (Med. Chem. Res. 1997, 7(1), 53-65) ;
. des analogues de la médétodimine (J. Med. Chem. 1992, 35(4), 750-5) ;
. 2-hydroxy-indane- (Bioorg. Med. Chem. 2000, 8(8), 1861-69) ;
. 2,3-dihydro-1H-indene (FR 2542738 et J. Med. Chem. 1988, 31(5), 944-48) ;
. 3,4-dihydro-2-naphthalene-methyl- et 2-naphthalene-methyl- et 2H-1-benzopyrane-methyl- et benzofurane-méthyl- et indène-méthyl- et indane-méthyl- (EP 1010693) ;
. quinoxaline- (ES 2009246) ;
. benzospiroalcène- (EP 0635497) ;
. bicyclo[4,2,0]-1,3,5-octatriène- (US 4567181).

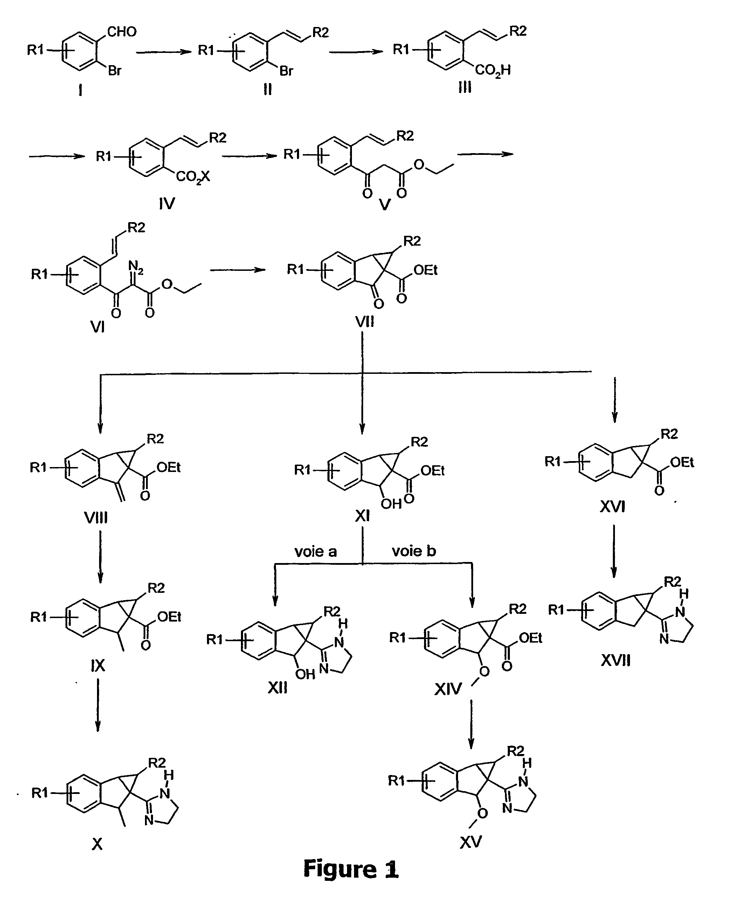

Il est remarquable, en outre, que certains des composés cités ci-dessus ne présentent que des différences structurales relativement minimes. Dans la publication J. Med. Chem. 2001, 44(5), 787-805, sont décrits des composés dont le squelette carboné est du type 1a,2,3,3a,7a,7b-hexahydro-1H-cyclopropa[a]naphtalène (figure a) : 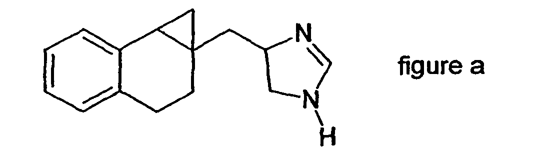 le poly-cycle carboné est lié a un hétérocycle du type 4-(4,5-dihydro-1H-imidazole) par l'intermédiaire d'un pont mono-méthylène (CH₂). L'ensemble des composés de l'invention ont un squelette carboné du type 1a,6-dihydro-1H-cyclopropa[a]indène directement lié à un hétérocycle du type 2-(4,5-dihydro-1H-imidazole). Ces différences structurales (i.e., taille du système polycyclique carboné, nombre de rotules intervenant dans la jonction carbocycle-hétérocycle, isomérie au niveau des hétérocycles azotés) induisent des différences significatives au niveau de leurs profils pharmacologiques. Par exemple, dans des conditions ordinaires de température, la mobilité conformationnelle inhérente à la structure des composés représentés par la figure a est très supérieure à celle des composés de l'invention. Il en découle des profils d'activité différents. C'est ainsi que, par exemple, les composés de l'invention interagissent de façon sélective avec les récepteurs alpha-2 adrénergiques alors que les composés décrits dans J. Med. Chem. 2001, 44(5), 787-805 interagissent aussi avec les sites de recapture de la sérotonine.
L'état de la technique le plus proche est représenté par des composés du type indanylimidazoles polycycliques (WO 0185698) répondant à la formule suivante (figure b) : 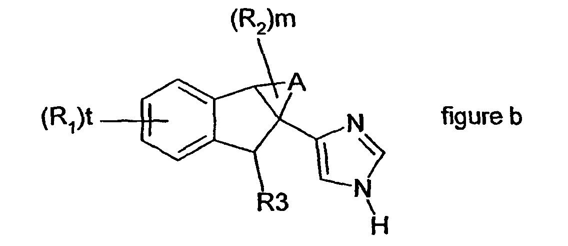 dans laquelle, entre autres :
- A peut former, avec les deux atomes de carbones par lesquels il est attaché, un mono-cycle carboné a 3 chaînons ;
- m peut être 0 ou 1;
- R2 peut être un groupe (C1-6)alcoyle
- t peut être 0 ou 1 ;
- t est 1 et R1 peut être un halogène ou un groupe (C1-6)alcoyloxy ;
- R3 peut être un hydrogène, OH, =O, (C1-6)alcoyle ou (C1-6)alcoyloxy ;
les composés représentés ci-dessus et les composés de la présente invention se différencient donc par la nature de leur hétérocycle azoté. A ce titre, il est connu de l'homme de l'art familier du domaine que la nature de l'hétérocycle azoté constitue un des déterminants majeurs du profil de l'activité pharmacologique du ligand. Compte tenu du grand nombre de structures faisant intervenir un motif 4,5-dihydro-1H-imidazole, déjà connus pour leur propriétés alpha-2 adrénergiques, il est surprenant que le motif 2-(1a,6-dihydro-1H-cyclopropa[a]inden-6a-yl)-4,5-dihydro-1H-imidazole confère aux composés de l'invention un profil pharmacologique tout à fait singulier. En effet, nous montrons, in vitro :
- une sélectivité des composés de l'invention vis à vis du sous-type alpha-2A humain versus alpha-2B humain. Cette caractéristique est importante puisque l'indication visée implique une action centrale du produit dans des structures cérébrales où les sites alpha-2B ne semblent pas distribués préférentiellement (Eur. J. Pharmacol. 1999, 366(1), 35-39 ; Nature 1999, 402, 181-83). Une interaction avec les sous-type alpha-2B tend donc à augmenter surtout la probabilité d'apparition d'effets indésirables.
- une activité intrinsèque du type agoniste inverse des composés de l'invention au niveau des récepteurs alpha-2A qui se distingue donc de celle des composés revendiqués dans WO 0185698.

De plus, nous montrons, in vivo, que les produits de l'invention sont capables de s'opposer à l'effet de la scopolamine dans un test de déficit mnésique considéré comme un modèle animal représentatif des troubles de la mémoire qui se manifestent au cours de la maladie d'Alzheimer (Psychopharmacology 1992, 106, 26-30 ; Exp. Neurol. 2000, 163, 495-529). Les composés de l'invention, dotés d'un tel profil d'activité, sont donc potentiellement utiles pour le traitement des maladies ou des troubles sensibles à l'action des agonistes partiels, des antagonistes ou des agonistes inverses des récepteurs alpha-2 adrénergiques, comme les maladies neurodégénératives pour lesquelles il existe un besoin thérapeutique important.

Enfin, le procédé de préparation des composés de l'invention est différent de celui des composés revendiqués dans WO 0185698 et met en oeuvre des intermédiaires de réaction nouveaux.
Plus spécifiquement, la présente invention a pour objet les nouveaux dérivés 2-(1a,6-dihydro-1H-cyclopropa[a]indén-6a-yl)-4,5-dihydro-1H-imidazole qui, sous forme de base, répondent à la formule générale (1) : 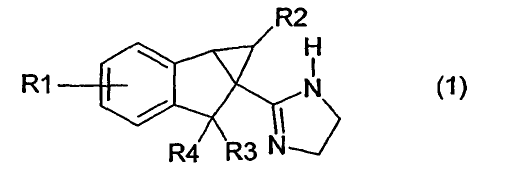 dans laquelle :
- R1 représente un atome d'hydrogène, un atome de fluor ou un groupe méthoxyle (OCH₃). Le substituant R1 sur le carbocycle aromatique peut occuper la position 2, 3, 4 ou 5 ;
- R2 représente un atome d'hydrogène ou un groupe méthyle ;
- R3 représente un atome d'hydrogène, un groupe méthyle, un groupe hydroxyle (OH) ou un groupe méthoxyle (OCH₃) ;
- R4 est un atome d'hydrogène ;
- R3 et R4 représentent ensemble un groupe carbonyle (C=O) ;
leurs sels d'addition et éventuellement les hydrates des sels d'addition avec les acides minéraux ou les acides organiques pharmaceutiquement acceptables ainsi que leurs formes tautomères, les énantiomères et les mélanges d'énantiomères et les stéréoisomères purs ou en mélange racémique ou non.

Dans un mode particulier de réalisation de l'invention, les composés de formule (1) dans laquelle :
- R1 et R2 ont la même signification que précédemment ;
- R3 représente un groupe méthyle, un groupe hydroxyle (OH) ou un groupe méthoxyle (OCH₃) ;
- R4 est un atome d'hydrogène ;
- les substituants R3 et 4,5-dihydro-1H-imidazole occupent des positions anti-périplanaires par rapport au plan défini par le noyau indanique ;
sont dans tous les cas les stéréoisomères préférés des produits de l'invention.

Dans un autre mode particulier de réalisation de l'invention, les composés de formule (1) dans laquelle :
- R1 a la même signification que précédemment ;
- R2 représente un groupe méthyle ;
- R3 représente un atome d'hydrogène, un groupe méthyle, un groupe hydroxyle (OH) ou un groupe méthoxyle (OCH₃) ;
- R4 est un atome d'hydrogène ;
- R3 et R4 représentent ensemble un groupe carbonyle (C=O) ;
- les substituants R2 et 4,5-dihydro-1H-imidazole occupent des positions syn-périplanaires par rapport au plan défini par le noyau cyclopropanique ;
sont dans tous les cas les stéréoisomères préférés des produits de l'invention.

Par anti-périplanaire, les inventeurs entendent les configurations relatives des molécules (1) pour lesquelles les substituants R3 et 4,5-dihydro-imidazole sont situés de part et d'autre du plan défini par le noyau indanique. Par syn-périplanaire, les inventeurs entendent les configurations relatives des molécules (1) pour lesquelles les substituants R2 et 4,5-dihydro-imidazole sont situés du même côté du plan défini par le noyau cyclopropanique.

Les composés de formule générale (1) peuvent exister sous plusieurs formes tautomères. De telles formes tautomères quoique non explicitement rapportées dans la présente demande pour simplifier la représentation graphique des formules développées sont néanmoins incluses dans le champ d'application de l'invention. Les composés de l'invention comportent plusieurs atomes de carbone asymétriques dans leur structure. De ce fait, ils existent sous la forme d'énantiomères et de diastéréoisomères. L'invention concerne aussi bien chaque stéréoisomère pur, c'est à dire associé avec moins de 5% d'un autre stéréoisomère ou d'un mélange d'autres stéréoisomères, que le mélange d'un ou plusieurs stéréoisomères en toutes proportions. Les composés de l'invention peuvent donc intervenir en tant que stéréoisomères purs ou mélanges racémiques ou non-racémiques de stéréoisomères.

L'invention s'étend enfin au procédé de préparation des dérivés de formule générale (1).

Les dérivés de formule générale (1) peuvent être obtenus par le procédé décrit dans le schéma illustré à l'annexe 1. La préparation des composés de l'invention utilise comme matière première les 2-bromo-benzaldéhydes appropriés, de formule (I), disponibles commercialement ou connus dans la littérature (i.e., RN 6630-33-7 ; RN 10401-18-0 ; RN 43192-31-0 ; RN 7507-80-0 ; RN 126712-07-0 ; RN 59142-68-6 ; RN 94569-84-3 ; RN 360575-28-6 ou préparer par réduction de l'acide correspondant RN 132715-69-6). Les dérivés du type 2-éthenyl- de formule (II-1) sont obtenus par une réaction de Wittig effectuée au moyen de bromure de méthyltriphénylphosphonium en milieu basique. Les dérivés du type 2-(1-propenyl)- de formule (II-2) et de stéréochimie (E) sont préparés sélectivement en 2 étapes selon la méthode décrite dans Tetrahedron 1995, 51(37), 10115-24 : addition de bromure d'éthylmagnésium sur la fonction aldéhyde suivi d'une réaction de déshydratation de l'alcool secondaire obtenu en milieu acide. L'introduction de la fonction acide carboxylique sur les dérivés de formule (II) est réalisée au moyen d'une réaction de brome-lithium échange suivie du piégeage de l'organolithien formé au moyen de CO₂. Les acides 2-ethenyl-benzoique (III-1) et (E)-2-(1-propenyl)-benzoïque (III-2) sont des composés connus dans la littérature (RN 27326-43-8 et RN 68692-67-1, respectivement). Les dérivés de formule (III), activés soit sous forme de chlorure d'acyle soit sous forme d'amide (IV), Synlett 1994, 2, 105-6, sont convertis en β-céto-esters (V) en appliquant une méthode analogue à celle décrite dans Synthesis 1993, 3, 290-92. L'intermédiaire clé dans la préparation des composés de l'invention est l'ester de l'acide 6-oxo-1a,6-dihydro-1H-cyclopropa[a]indène-6a-carboxylique de formule (VII). Ce dernier est obtenu par l'addition intramoléculaire d'un carbénoïde sur la double liaison selon Doyle, M.P. ; McKervey, M.A. ; Ye, T. Modern Catalytic Methods for Organic Synthesis with Diazo Compounds, John Wiley & Sons, Inc. 1998 chapitre 5, pages 238-288. Le carbénoïde est obtenu par la décomposition d'un précurseur du type diazo (VI), lui-même préparé à partir des esters des acides 3-oxo-3-(2-ényl-aryl)-propioniques de formule (V) selon la méthode décrite dans Synth. Commun. 1987, 17(4), 1709-16.

A partir du composé de formule (VII) nous préparons l'ensemble des composés de l'invention. Ainsi, les composés (X) dans lesquels : R1 est H, F ou OCH₃ ; R2 est H ou CH₃; R3 est CH₃ et R4 est H sont obtenus par : méthylénation de (VII) au moyen de bromure de méthyltriphénylphosphonium en présence d'une base selon une réaction de Wittig dassique ; réduction de la double liaison exo-cyclique formée au moyen du diimide selon une procédure analogue à celle décrite dans Tetrahedron 1976, 32, 2157-62 et condensation d'éthylènediamine en présence de triméthylaluminium sur les esters (IX) selon une technique décrite dans J. Org. Chem. 1987, 46, 2824-26.

Les composé de formule (XII, XIII et XV) dans lesquels : R1 est H, F ou OCH₃ ; R2 est H ou CH₃ ; R3 est OH, OCH₃ ; R4 est H ou R3 et R4 forment ensemble un groupe carbonyle (=O) sont, eux aussi, préparés à partir de l'intermédiaire (VII). Ainsi, la fonction 6-oxo de (VII) peut-être réduite en l'alcool (XI) au moyen, par exemple, d'un donneur d'hydrure. La réduction par du borohydrure de sodium dans l'éthanol à froid est diastéréosélective ; l'isomère dans lequel les groupes hydroxyle (OH) et cyclopropane occupent des positions syn-périplanaire par rapport au plan défini par le noyau indanique est le seul observé dans la réaction en question. L'alcool secondaire (XI) peut-être ensuite soit directement converti en l'hétérocycle attendu (XII), voie a, annexe 1 ; soit méthylé en l'éther (XIV) puis converti en l'hétérocycle attendu (XV), voie b, annexe 1. L'oxydation des alcools de formule (XII) fourni les composés de formule (XIII).

Les composé de formule (XVI) dans lesquels : R1 est H, F ou OCH₃ ; R2 est H ou CH₃ ; R3 et R4 sont H proviennent de la réduction complète de la fonction 6-oxo du composé de formule (VII) selon une méthode analogue à celle décrite dans J. Org. Chem. 1973, 38(15), 2675-81. L'hétérocycle azoté contenu dans (XVII) est ensuite formé comme décrit précédemment.

Les composés de formule (X), (XII), (XIII), (XV) et (XVII) constituent l'ensemble des composés de l'invention.

L'invention a aussi pour objet les compositions pharmaceutiques contenant à titre de principe actif au moins un des dérivés de formule générale (1) ou un de ses sels ou hydrates de ses sels en combinaison avec un ou plusieurs support inertes ou autres véhicules pharmaceutiquement acceptables.

Les compositions pharmaceutiques selon l'invention peuvent être, à titre d'exemple, des compositions administrables par voie orale, nasale, sublinguale, rectale ou parentérale. A titre d'exemple de compositions administrables par voie orale on peut citer les comprimés, les gélules, les granules, les poudres et les solutions ou suspensions orales.

Les formulations appropriées pour la forme d'administration choisie sont connues et décrites, par exemple dans : Remington, The Science and Practice of Pharmacy, 19^{ème} édition, 1995, Mack Publishing Company.

La dose efficace d'un composé de l'invention varie en fonction de nombreux paramètres tels que, par exemple, la voie d'administration choisie, le poids, l'âge, le sexe, l'état d'avancement de la pathologie à traiter et la sensibilité de l'individu à traiter. En conséquence, la posologie optimale devra être déterminée, en fonction des paramètres jugés pertinents, par le spécialiste en la matière. Bien que les doses efficaces d'un composé de l'invention puissent varier dans de larges proportions, les doses journalières pourraient s'échelonner entre 0,01 mg et 100 mg par Kg de poids corporel de l'individu à traiter. Une dose journalière d'un composé de l'invention comprise entre 0,10 mg et 50 mg par Kg de poids corporel de l'individu à traiter étant, toutefois, préférée.

Les compositions pharmaceutiques selon l'invention sont utiles dans le traitement des maladies neurodégénératives.

### Exemples

Les exemples suivants illustrent l'invention mais ne la limitent en aucune façon.

Dans les exemples et les exemples de référence ci-après :
(i) l'avancement des réactions est suivi par chromatographie sur couche mince (CCM) et par conséquent les temps de réaction ne sont mentionnés qu'à titre indicatif ;
(ii) des formes cristallines différentes peuvent donner des points de fusion différents, les points de fusion rapportés dans la présente demande sont ceux des produits préparés selon la méthode décrite et ne sont pas corrigés ;
(iii) la structure des produits obtenus selon l'invention est confirmée par les spectres de résonance magnétique nucléaire (RMN), infrarouge (IR) et l'analyse centésimale, la pureté des produits finaux est vérifiée par CCM, la pureté énantiomérique des intermédiaires réactionnels et des produits finaux est déterminée par HPLC sur phase chirale;
(iv) les spectres RMN sont enregistrés dans le solvant indiqué. Les déplacements chimiques (δ) sont exprimés en partie par million (ppm) par rapport au tétraméthylsilane. La multiplicité des signaux est indiquée par : s, singulet ; d, doublet ; t, triplet ; q, quadruplet; m, multiplet ; 1, large ;
(v) les différents symboles des unités ont leur signification habituelle : µg (microgramme) ; mg (milligramme) ; g (gramme) ; ml (millilitre) ; mV (millivolt) ; °C (degré Celsius) ; mmole (millimole) ; nmole (nanomole) ; cm (centimètre) ; nm (nanomètre) ; min (minute) ; ms (milliseconde), Hz (hertz) ; [α] (pouvoir rotatoire spécifique mesuré à 589 nm, 25°C et à la concentration c, dans la présente invention la dimension deg cm² g⁻¹ est toujours sous entendue) ; les pressions sont données en millibars (mb) ;
(vi) les abréviations ont la signification suivante : F (point de fusion) ; Eb (point d'ébullition) ; AUC (aire sous la courbe) ;
(vii) par "température ambiante" on entend une température comprise entre 20°C et 25°C.

### Exemple 1 : 6-oxo-1a,6-dihydro-1H-cyclopropa[a]indèn-6a-carboxylate d'éthyle (VII-1)

Dans la solution de 33,42 g (0,153 mole) d'ortho-vinylbenzoylacétate d'éthyle (V-1), 36,76 g (0,153 mole) de para-acétamidobenzènesulfonylazide et 300 ml de THF anhydre sous agitation sur bain de glace et sur azote, on ajoute goutte à goutte la solution de 23,06 ml (0,153 mole) de DBU dans 35 ml de THF anhydre. Après 16 heures d'agitation à température ambiante, la solution rouge-grenat est versée dans un mélange de solution aqueuse saturée de NH₄Cl et de glace. Le mélange est extrait 2 fois par l'acétate d'éthyle. Les phases organiques sont décantées, lavées à l'eau puis à l'eau salée. Après, séchage sur MgSO₄ et filtration, le solvant est évaporé sous vide (température inférieure à 40°C). La masse cristalline obtenue est reprise dans un mélange 50 : 50 cyclohexane/acétate d'éthyle et le para-acétamidobenzène- sulfonylamide est filtré. Les eaux-mères sont amenées à sec sous vide (T° < 40°C). L'huile brune obtenue est purifiée par filtration rapide sur 200 g de silice en utilisant le CH₂Cl₂ comme solvant. Après élimination du solvant (T° < 40°C), on obtient 35,81 g (95,8 %) d'une huile orangée utilisée directement dans la réaction de cycloaddition.

Dans la suspension de 1,18 g d'acétate de Rhodium et 250 ml de CH₂Cl₂ anhydre, sous agitation à température ambiante, on introduit goutte à goutte la solution du diazoacétate (VI-1) obtenu précédemment dans 50 ml de CH₂Cl₂ anhydre. Après une nuit d'agitation à température ambiante, le catalyseur est filtré et le solvant est éliminé sous vide. Le produit brut est purifié par chromatographie sur 320 g de silice en utilisant le cyclohexane à 10 % d'acétate d'éthyle comme solvant.

On obtient le produit du titre (23,9 g) ;
Rendement : 75,7 %
C₁₃H₁₂O₃ : 216,24
IR (film) ν : 1720 et 1746 cm⁻¹ (C=O)
¹H RMN (CDCl₃) : 1,33 (t, 3H) ; 1,74 (t, 1H) ; 2,39 (dd, 1H) ; 3,37 (dd, 1H) ; 4,29 (q, 2H) ; 7,35 (t, 1H) ; 7,45 (d, 1H) ; 7,51 (t, 1H) ; 7,70 (d, 1H)
¹³C RMN (CDCl₃) : 14,13 ; 32,04 ; 38,61 ; 39,77 ; 61,56 ; 124,43 ; 125,31 ; 127,63 ; 134,11 ; 134,13 ; 151,46 ; 168,50 ; 195,44.

### Exemple 2 : 1a,6-dihydro-1H-cyclopropa[a]indén-6a-carboxylate d'éthyle (XVI-1)

Dans la solution de 0,92 g (4,25 mmoles) de (VII-1) dans 4 ml d'acide trifluoroacétique, sous agitation, sous azote et sur bain de glace, on ajoute goutte à goutte 1,7 ml (10,64 mmoles) de triéthylsilane. Après 4 heures d'agitation à température ambiante la solution est versée dans un mélange glace/eau. Après addition d'acétate d'éthyle, le mélange est alcalinisé par addition de bicarbonate de sodium sous agitation énergique. La phase organique est décantée, lavée à l'eau puis à l'eau salée. Après séchage sur MgSO₄ et filtration, l'huile brute obtenue est purifiée par chromatographie sur silice en utilisant le cyclohexane à 2 % d'acétate d'éthyle comme éluant. On obtient le produit du titre (0,47 g) ;

Rendement : 54,6 %
C₁₃H₁₄O₂ : 202,24
IR (film) ν : 1721 cm⁻¹ (C=O)
¹H RMN (CDCl₃) : 0,68 (t, 1H) ; 1,27 (t, 3H) ; 1,98 (dd, 1H) ; 2,95 (ddd, 1H) ; 3,06 (d, 1H) ; 3,72 (d, 1H) ; 4,18 (q, 2H) ; 7,13 (m, 2H) ; 7,18 (m, 1H) ; 7,27 (m, 1H).

### Exemple 3 : 1-exo-Méthyl-1a,6-dihydro-1H-cyclopropa[a]indén-6a-carboxylate d'éthyle (XVI-2)

C₁₄H₁₆O₂ : 216,28
IR (film) ν : 1717 cm⁻¹ (C=O)
¹H RMN (CDCl₃) : 1,04 (m, 1H) ; 1,29 (t, 3H) ; 1,35 (d, 3H) ; 2,77 (d, 1H, J = 4,4Hz) ; 3,14 (d, 1H) ; 3,58 (d, 1H) ; 4,21 (m, 2H) ; 7,11 (m, 3H) ; 7,24 (m, 1H).

### Exemple 4 : 6-Méthylène-1a,6-dihydro-1H-cyclopropa[a]indén-6a-carboxylate d'éthyle (VIII)

Dans la suspension de 7,5 g (21 mmoles) de bromure de méthyltriphénylphosphonium et 45 ml de THF anhydre sous agitation à température ambiante, on ajoute par fractions 2,56 g (21 mmoles) de tBuOK. La suspension est maintenue sous agitation pendant 1h 30 puis sur bain de glace introduit goutte à goutte la solution de 3,02 g (14 mmoles) de (VII-1) et 5 ml de THF anhydre. Après une nuit d'agitation à température ambiante, la suspension est versée dans une solution aqueuse saturée de NH₄Cl et extraite 2 fois par l'acétate d'éthyle. Les phases organiques sont lavées à l'eau puis à l'eau salée. Après séchage sur MgSO₄ et filtration le solvant est éliminé sous vide. Le triphénylphosphine oxyde est cristallisé dans l'éther isopropylique et les eaux-mères sont amenées à sec sous vide. L'huile résiduelle est purifiée par chromatographie sur silice en utilisant le cyclohexane à 3 % d'acétate d'éthyle comme éluant. On obtient le produit du titre (1,8 g) ;
Rendement : 59 %
C₁₄H₁₄O₂ : 214,25
¹H RMN (CDCl₃) : 1,04 (t, 1H) ; 1,30 (t, 3H) ; 2,16 (dd, 1H) ; 3,16 (dd, 1H) ; 4,21 (m, 2H) ; 5,73 (s, 1H) ; 5,84 (s, 1H) ; 7,19 (m, 2H) ; 7,30 (m, 1H) ; 7,47 (m, 1H).

### Exemple 5: 6-Méthyl-1a,6-dihydro-1H-cyclopropa[a]indén-6a-carboxylate d'éthyle (IX)

Dans la suspension de 2,8 g (13 mmoles) de (VIII), 5,6 g (26 mmoles) de 2, 4, 6 - triméthylbenzènesulfonylhydrazide et 20 ml de méthanol anhydre, sous agitation à température ambiante, on ajoute goutte à goutte 3,65 ml (26 mmoles) de triéthylamine puis on porte le mélange au reflux pendant 5 heures. Le méthanol est éliminé sous vide; on ajoute une solution aqueuse saturée de NaHCO₃ à 10 % et extrait 2 fois par l'éther. Les phases organiques sont lavées à l'eau salée, séchées sur MgSO₄, filtrées et amenées à sec sous vide. L'huile résiduelle est purifiée par chromatographie sur silice en utilisant le cyclohexane à 50 % de dichlorométhane comme éluant. On obtient le produit du titre (2,19 g) ;
Rendement : 78%
C₁₄H₁₆O₂ : 216,27
¹H RMN (CDCl₃) : 0,64 (t, 1H) ; 1,27 (t, 3H) ; 1,36 (d, 3H) ; 1,77 (dd, 1H) ; 2,93 (dd, 1H) ; 4,04 (q, 1H) ; 4,19 (q, 2H) ; 7,14 (m, 3H) ; 7,25 (d, 1H).

### Exemple 6: 6-Hydroxy-1a,6-dihydro-1H-cyclopropa[a]indén-6a-carboxylate d'éthyle (XI-1)

Dans la solution de 5,25 g (24,3 mmoles) de (VII-1) dans 50 ml d'éthanol absolu, sous agitation sur bain de glace, on ajoute par fractions 1,97 g (36,4 mmoles) de KBH₄ puis maintient sous agitation une nuit en laissant le bain remonter à température ambiante. L'ethanol est éliminé sous vide, le résidu est repris par un mélange glace/eau et extrait 2 fois par l'acétate d'éthyle. Les phases organiques sont lavées à l'eau salée, séchées sur MgSO₄, filtrées et amenées à sec sous vide. Le résidu est purifié par chromatographie sur silice en utilisant le cyclohexane à 20 % d'acétate d'éthyle comme éluant. On obtient le composé du titre (4,98 g) ;
Rendement : 95 %
C₁₃H₁₄O₃ : 218,24
IR (film) ν : 3440 cm⁻¹(OH) ; 1720 cm⁻¹ (C=O)
¹H RMN (CDCl₃) : 1,67 (t, 1H) ; 1,29 (t, 3H) ; 1,83 (dd, 1H) ; 2,34 (d, 1H) ; 3,01 (dd, 1H) ; 4,22 (m, 2H) ; 6,03 (d, 1H) ; 7,22 (m, 3H) ; 7,31 (m, 1H).

### Exemple 7: 6-Méthoxy-1a,6-dihydro-1H-cyclopropa[a]indén-6a-carboxylate d'éthyle (XIV-1)

Dans la solution de 5 g (22,9 mmoles) de (XI-1) et 80 ml d'acétonitrile anhydre sous agitation à température ambiante. On ajoute 26,53 g (114,5 mmoles) d'Ag₂O puis goutte à goutte rapidement, 7,13 ml (114,5 mmoles) d'iodure de méthyle. On maintient la suspension sous agitation à température ambiante, à l'abri de la lumière pendant 48 heures. La suspension est filtrée et le solvant éliminé sous vide. L'huile résiduelle est purifiée par chromatographie sur silice en utilisant le cyclohexane à 5 % d'acétate d'éthyle comme éluant. On obtient le composé du titre (4,3 g) ;
Rendement : 81 %
C₁₄H₁₆O₃ : 232,27
¹H RMN (CDCl₃) : 1,29 (m, 4H) ; 1,92 (dd, 1H) ; 2,97 (dd, 1H) ; 3,57 (s, 3H) ; 4,21 (m, 2H) ; 5,73 (s, 1H) ; 7,20 (m, 3H) ; 7,28 (m, 1H).

### Exemple 8: 2-(1a,6-Dihydro-1H-cyclopropa[a]indén-6a-yl)-4,5-dihydro-1H-imidazole (XVII-1)

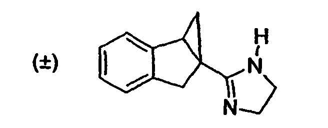

Dans la solution de 1,5 ml (3 mmoles) de solution toluénique de triméthylaluminium 2 M et 10 ml de toluène anhydre sous agitation énergique à -10°C, on introduit goutte à goutte 0,23 ml (3,45 mmoles) d'éthylène diamine. On maintient sous agitation à température ambiante pendant 30 minutes puis goutte à goutte la solution de 0,47 g (2,3 mmoles) de (XVI-1) dans 2 ml de toluène anhydre. Le mélange est porté au reflux que l'on maintient pendant 2 heures. Sur bain de glace sous agitation énergique on ajoute lentement 1,3 ml d'eau et maintient 30 minutes à température ambiante. La phase organique est décantée, diluée par l'acétate d'éthyle, lavée à l'eau salée, séchée (Na₂SO₄) et amenée à sec sous vide. Le produit brut est purifié par chromatographie sur alumine en utilisant le dichlorométhane à 2 % de méthanol. On obtient le produit du titre (0,31 g) ; Rendement : 67 %
C₁₃H₁₄N₂ : 198,26
¹H RMN (CDCl₃) : 0,70 (t, 1H) ; 1,51 (dd, 1H) ; 2,89 (dd, 1H) ; 3,20 (d, 1H) ; 3,62 (d, 1H) ; 3,66 (s, 4H) ; 7,12 (m, 2H) ; 7,17 (m, 1H) ; 7,25 (m, 1H).

Oxalate du composé du titre :
F : 164-166°C
C₁₅H₁₆N₂O₄ : 288,29
Calculé % : C 62,49, H 5,59, N 9,72
Trouvé % : C 62,46, H 5,72, N 9,66
¹H RMN (DMSOd₆) : 0,93 (t, 1H) ; 2,03 (dd, 1H) ; 3,24 (d, 1H) ; 3,29 (dd, 1H) ; 3,54 (d, 1H) ; 3,83 (s, 4H) ; 7,18 (m, 2H) ; 7,25 (m, 1H) ; 7,36 (m, 1H).

Le dédoublement des composés de formule (XVII-1) est effectué par séparation chromatographique des céto-esters diastéréoisomères (VII-3) de (R)-(-)-pantolactone (RN 599-04-2) :

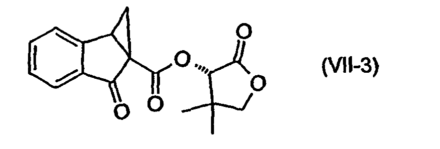

### Exemple 9: (+)-2-(1a,6-Dihydro-1H-cyclopropa[a]indén-6a-yl)-4,5-dihydro-1H-imidazole (+)-(XVII-1)

Chlorhydrate du composé du titre :
F : 245-247°C
C₁₃H₁₅ClN₂ : 234,73
Calculé % : C 66,52, H 6,44, N 11,93
Trouvé % : C 66,34, H 6,65, N 11,71
¹H RMN (D₂O) : 1,01 (t, 1H) ; 1,92 (dd, 1H) ; 3,26 (m, 1H) ; 3,34 (d, 1H) ; 3,47 (d, 1H) ; 3,93 (s, 4H) ; 7,28 (m, 2H) ; 7,32 (m, 1H) ; 7,42 (m, 1H).
[α]²⁵_{D}: + 218,2°C (c = 0,369, méthanol).

### Exemple 10 : (-)-2-(1a,6-Dihydro-1H-cyclopropa[a]indén-6a-yl)-4,5-dihydro-1H-imidazole (-)-(XVII-1)

Chlorhydrate du composé du titre :
F : 245-247°C
C₁₃H₁₅ClN₂ : 234,73
Calculé % : C 66,52, H 6,44, N 11,93
Trouvé % : C 65,85, H 6,45, N 11,69
¹H RMN (D₂O) : 1,01 (t, 1H) ; 1,92 (dd, 1H) ; 3,26 (m, 1H) ; 3,34 (d, 1H) ; 3,47 (d, 1H) ; 3,93 (s, 4H) ; 7,28 (m, 2H) ; 7,32 (m, 1H) ; 7,42 (m, 1H).
[α]²⁵_{D} : - 219,9° (c = 0,467, méthanol).

### Exemple 11: 2-(1-exo-Méthyl-1a,6-dihydro-1H-cyclopropa[a]indén-6a-yl)-4,5-dihydro-1H-imidazole (XVII-2)

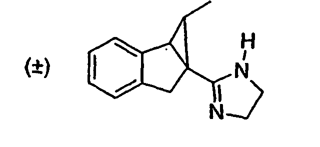

En procédant comme dans l'exemple 8 mais en utilisant le composé (XVI-2) à la place du composé de formule (XVI-1) on obtient le composé du titre.
Rendement : 30 %
C₁₄H₁₆N₂ : 212,28
¹H RMN (CDCl₃) : 0,88 (m, 1H) ; 1,22 (d, 3H) ; 2,57 (d, 1H) ; 3,19 (d, 1H) ; 3,35 (d, 1H) ; 3,63 (s, 4H) ; 7,10 (m, 3H) ; 7,25 (d, 1H).

Fumarate du composé du titre :
F : 133-135°C
C₁₈H₂₀N₂O₄ : 328,37
Calculé % : C 65,84, H 6,14, N 8,53
Trouvé % : C 65,51, H 6,35, N 8,65
¹H RMN (DMSOd₆) : 1,08 (m, 1H) ; 1,17 (d, 3H) ; 3,15 (d, 1H) ; 3,29 (d, 1H) ; 3,35 (d, 1H) ; 3,82 (s, 4H) ; 6,43 (s, 2H) ; 7,13 (m, 2H) ; 7,19 (m, 1H) ; 7,32 (m, 1H).

### Exemple 12: 2-(6-Méthyl-1a,6-dihydro-1H-cyclopropa[a]indén-6a-yl)-4,5-dihydro-1H-imidazole (X)

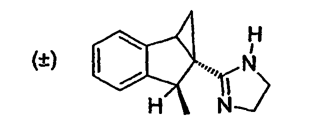

En procédant comme dans l'exemple 8 mais en utilisant le composé (IX) à la place du composé de formule (XVI-1) on obtient le composé du titre.
Rendement : 68 %
C₁₄H₁₆N₂ : 212,28
¹H RMN (CDCl₃) : 0,65 (t, 1H) ; 1,40 (d, 3H) ; 1,63 (dd, 1H) ; 2,81 (dd, 1H) ; 3,65 (s, 4H) ; 3,97 (q, 1H) ; 7,16 (m, 3H) ; 7,23 (m, 1H).

Chlorhydrate du composé du titre :
sublimation 250°C
C₁₄H₁₇ClN₂ : 248,76
Calculé % : C 67,60, H 6,89, N 11,26
Trouvé % : C 67,18, H 6,96, N 11,04
¹H RMN (D₂O) : 0,97 (t, 1H) ; 1,38 (d, 3H) ; 1,75 (dd, 1H) ; 3,15 (dd, 1H) ; 3,82 (q, 1H) ; 3,92 (s, 4H) ; 7,27 (m, 3H) ; 7,31 (m, 1H).

### Exemple 13 : 6a-(4,5-Dihydro-1H-imidazol-2-yl)-1,1a,6,6a-tetrahydro-cyclopropa[a]indén-6-β-ol (XII-1)

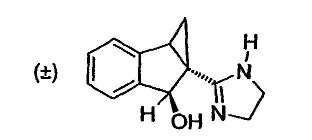

En procédant comme dans l'exemple 8 mais en utilisant le composé (XI-1) à la place du composé de formule (XVI-1) on obtient le composé du titre.
Rendement : 27 %
C₁₃H₁₄N₂O : 214,26
¹H RMN (DMSOd₆) : 1,29 (t, 1H) ; 1,85 (dd, 1H) ; 3,19 (dd, 1H) ; 3,81 (s, 4H) ; 5,76 (s, 1H) ; 7,22 (m, 2H) ; 7,28 (m, 2H).

Chlorhydrate du composé du titre :
F : 229-231°C
C₁₃H₁₅ClN₂O : 250,73
Calculé % : C 62,28, H 6,03, N 11,17
Trouvé % : C 62,36, H 6,05, N 11,29
¹H RMN (D₂O) : 1,41 (t, 1H) ; 1,84 (dd, 1H) ; 3,30 (dd, 1H) ; 3,97 (s, 4H) ; 5,85 (s, 1H) ; 7,36 (m, 4H).

### Exemple 14: 2-(6-Méthoxy-1a,6-dihydro-1H-cyclopropa[a]indén-6a-yl)-4,5-dihydro-1H-imidazole (XV-1)

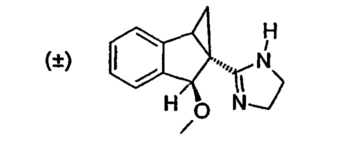

En procédant comme dans l'exemple 8 mais en utilisant le composé (XIV-1) à la place du composé de formule (XVI-1) on obtient le composé du titre.
Rendement : 51 %
C₄H₁₆N₂O : 228,28
¹H RMN (CDCl₃) : 1,25 (t, 1H) ; 1,66 (dd, 1H) ; 2,75 (dd, 1H) ; 3,59 (s, 3H) ; 3,64 (s, 4H) ; 5,61 (s, 1H) ; 7,18 (m, 3H) ; 7,27 (m, 1H).

Fumarate du composé du titre :
F : 173-175°C
C₁₈H₂₀N₂O₅ : 344,37
Calculé % : C 62,78, H 5,85, N 8,13
Trouvé % : C 62,64, H 5,94, N 8,15
¹H RMN (DMSOd₆) : 1,18 (t, 1H) ; 2,09 (dd, 1H) ; 3,08 (dd, 1H) ; 3,44 (s, 3H) ; 3,75 (s, 4H) ; 5,68 (s, 1H) ; 6,44 (s, 2H) ; 7,17-7,31 (m, 4H).

Le dédoublement est effectué par séparation chromatographique sur support chiral (CHIRALPACK AD, éluant : Hexane à 5% de méthanol).

### Exemple 15 : (+)-2-(6-Méthoxy-1a,6-dihydro-1H-cyclopropa[a]indén-6a-yl)-4,5-dihydro-1H-imidazole (+)-(XV-1)

Fumarate du composé du titre :
F : 170-172°C
C₁₈H₂₀N₂O₅ : 344,37
Calculé % : C 62,78, H 5,85, N 8,13
Trouvé % : C 62,55, H 5,89, N 8,05
¹H RMN (DMSOd₆) : 1,19 (t, 1H) ; 2,09 (dd, 1H) ; 3,09 (dd, 1H) ; 3,44 (s, 3H) ; 3,75 (s, 4H) ; 5,67 (s, 1H) ; 6,44 (s, 2H) ; 7,19-7,31 (m, 4H)
   [α]²⁵_{D} : + 174,15° (c = 0,16, méthanol).

### Exemple 16 : (-)-2-(6-Méthoxy-1a,6-dihydro-1H-cyclopropa[a]indén-6a-yl)-4,5-dihydro-1H-imidazole (-)-(XV-1)

Fumarate du composé du titre :
F : 170-172°C
C₁₈H₂₀N₂O₅ : 344,37
Calculé % : C 62,78, H 5,85, N 8,13
Trouvé % : C 62,45, H 5,92, N 8,05
¹H RMN (DMSOd₆) : 1,19 (t, 1H) ; 2,09 (dd, 1H) ; 3,09 (dd, 1H) ; 3,44 (s, 3H) ; 3,75 (s, 4H) ; 5,67 (s, 1H) ; 6,44 (s, 2H) ; 7,19-7,31 (m, 4H)
[α]²⁵_{D} : - 164,16° (c = 0,14, méthanol).

### Exemple 17: 2-(2,6-Diméthoxy-1a,6-dihydro-1H-cyclopropa[a]indén-6a-yl)-4,5-dihydro-1H-imidazole (XV-1a)

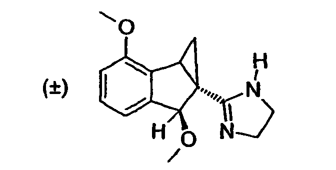

En procédant comme dans l'exemple 8 mais en utilisant le composé (XIV-1a) à la place du composé de formule (XVI-1) on obtient le composé du titre.
Rendement : 55 %
C₁₅H₁₈N₂O₂ : 258,31
¹H RMN (CDCl₃) : 1,31 (t, 1H) ; 1,68 (dd, 1H) ; 2,84 (dd, 1H) ; 3,58 (s, 3H) ; 3,63 (s, large, 4H) ; 3,83 (s, 3H) ; 5,68 (s, 1H) ; 6,73 (d, 1H) ; 6,90 (d, 1H) ; 7,14 (t, 1H).

Fumarate du composé du titre :
F : 177-179°C
C₁₉H₂₂N₂O₆ : 374,39
Calculé % : C 60,95, H 5,92, N 7,48
Trouvé % : C 60,24, H 6,39, N 7,08
¹H RMN (DMSOd₆): 1,19 (t, 1H) ; 2,03 (dd, 1H) ; 3,05 (dd, 1H) ; 3,43 (s, 3H) ; 3,74 (s, 4H) ; 3,80 (s, 3H) ; 5,64 (s, 1H) ; 6,45 (s, 2H) ; 6,86 (m, 2H) ; 7,19 (t, 1H).

### Exemple 18 : 2-(3-Fluoro-6-methoxy-1a,6-dihydro-1H-cyclopropa[a]inden-6a-yl)-4,5-dihydro-1H-imidazole (XV-1b)

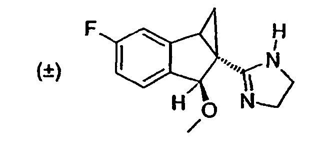

En procédant comme dans l'exemple 8 mais en utilisant le composé (XIV-1b) à la place du composé de formule (XVI-1) on obtient le composé du titre.

### Exemple 19 : 2-(3-Fluoro-1-exo-méthyl-1a,6-dihydro-1H-cyclopropa[a]inden-6a-yl)-4,5-dihydro-1H-imidazole(XVII-2b)

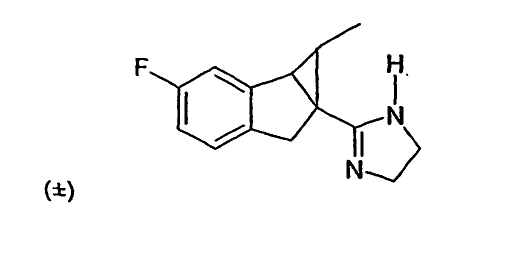

En procédant comme dans l'exemple 8 mais en utilisant le composé (XVI-2) à la place du composé de formule (XVI-1) on obtient le composé du titre.

Les composés de formule (1) ainsi que leurs sels thérapeutiquement acceptables présentent des propriétés pharmacologiques intéressantes.

Les résultats des essais sont regroupés dans le tableau suivant :

| **Composé** | **Affinité** (pKi) | | **Activité Intrinsèque** | **Déficit mnésique à la scopolamine** |
|---|---|---|---|---|
| | Alpha-2A | Alpha-2B | % stimulé | amplitude de l'effet % (dose, mg/kg i.p.) |
| XV-1 | 7,9 | 6,5 | -2 | +168 (2,5) |
| (+)-XV-1 | 8,4 | <5 | -63 | - |
| XVII-2 | 8,5 | 7,7 | -64 | +181 (0,63) |
| (-)-adrénaline | - | - | +100 | - |
| donepezil | - | - | - | +67 (0,16) |

### Liaisons aux récepteurs alpha-2 adrénergiques :

Les membranes des cellules C6 exprimant de façon permanente soit le récepteur humain alpha-2A soit alpha-2B sont préparés dans du Tris-HCl (pH = 7,6). Les essais de liaison sont effectués avec 2 nM [³H]RX 821002. Le milieu d'incubation est composé de 0,4 ml de membranes cellulaires (10 µg de protéines), 0,05 ml de radioligand et 0,05 ml de produit à tester ou de phentolamine (10 µM) pour déterminer la liaison non spécifique. La réaction est arrêtée après 30 minutes d'incubation à 25°C en ajoutant 3 ml de Tris-HCl, 50 mM (pH = 7,6), froid, suivie d'une filtration sur filtres Whatman, GF/B à l'aide d'un Brandel. Les valeurs du Ki sont calculée selon l'équation Ki = IC₅₀/(1 + C/Kd) où C est la concentration et Kd la constante de dissociation, pKi = - logKi. Dans ces conditions, il apparaît que les composés de l'invention possèdent une forte affinité pour les récepteurs du sous type alpha-2A adrénergiques humains alors qu'ils n'ont qu'une faible, voire aucune, affinité pour les récepteurs du sous type alpha-2B adrénergiques humains (cf. tableau ci-dessus). Cette sélectivité réceptorielle inattendue des composés de l'invention, comparée aux composés de l'art antérieur, peut avoir un impact favorable sur leur tolérance.

### Mesure de l'activation des récepteurs alpha-2 adrénergiques :

Les réponses en GTPγS sont effectuées sur des préparations membranaires dans l'HEPES 20 mM (pH = 7,4) avec 30 µM de GDP, 100 mM de NaCl, 3 mM de MgCl₂ et 0,2 mM d'acide ascorbique. La stimulation maximale du GTPγS est déterminée en présence de 10 mM de (-)-adrénaline et calculée versus la réponse GTPγS basale. Les résultats sont exprimés versus soit l'adrénaline soit du RX 811059. Dans ces conditions, les composés de l'invention se distinguent de la plupart des composés de l'art antérieur du type 4,5-dihydro-1H-imidazole et/ou 1H-imidazole en ce qu'ils se comportent plutôt comme des agonistes inverses au niveau des récepteurs adrénergiques alpha-2A humains (cf. tableau ci-dessus).

### Test du déficit mnésique induit par la scopolamine :

La scopolamine possède des propriétés amnésiantes chez l'animal et l'homme. Ainsi, son administration chez l'homme sain provoque certains symptômes proches de ce qui est observé dans la maladie d'Alzheimer. Le déficit mnésique induit par la scopolamine est donc utilisé comme modèle pharmacologique expérimental de cette pathologie. La scopolamine réduit la capacité d'acquisition, de mémorisation et de rappel dans un test d'évitement passif chez le rat. Il s'agit de mesurer la réticence, après apprentissage, qu'éprouve un animal à entrer dans un compartiment sombre où il reçoit un choc électrique de faible intensité. L'administration de scopolamine supprime cette réticence, et les composés étudiés s'opposent à l'effet de la scopolamine. Le protocole expérimental utilisé est décrit dans Psychopharmacol. 1992, 106, 26-30.

Les composés de l'invention montrent une activité importante dans ce test (cf. tableau ci-dessus). L'amplitude de l'effet obtenu avec les composés de l'invention est supérieur à celui, par exemple, du donézépil, inhibiteur d'acétylcholinestérase utilisé en clinique pour le traitement de la maladie d'Alzheimer (Chem. Rec. 2001, 1(1), 63-73). Les composés de l'invention sont donc capables de s'opposer efficacement au déficit mnésique induit par la scopolamine .

Les résultats des essais montrent donc que les composés de formule (1) :
- possèdent une forte affinité pour les récepteurs adrénergiques du sous type alpha-2A humains ;
- n'ont pas d'affinité ou une faible affinité pour les récepteurs adrénergiques alpha-2B humains ;
- se comportent, généralement, comme des agonistes inverses au niveau des récepteurs adrénergiques alpha-2A humains ;
- sont actifs, in vivo, dans un modèle animal considéré comme représentatif des troubles de la mémoire qui se manifestent au cours de la maladie d'Alzheimer.

De ce fait, les composés de l'invention ainsi que leurs sels thérapeutiquement acceptables sont potentiellement utiles comme médicaments, en particulier dans le traitement de certaines pathologies neurodégénératives progressives telle que, par exemple, la maladie d'Alzheimer.

L'administration des composés de l'invention peut être réalisée par voie orale, nasale, sublinguale, rectale ou parentérale. A titre d'exemples de formulation non limitatifs, nous donnons ci-après, une préparation des composés de l'invention. Les ingrédients ainsi que d'autres, thérapeutiquement acceptables, peuvent être introduits en d'autres proportions sans modifier la portée de l'invention. Les termes 'ingrédient actif' utilisés dans l'exemple de formulation ci-après font références à un composé de formule (1) ou un sel d'addition ou éventuellement un hydrate d'un sel d'addition du composé de formule (1) avec un acide minéral ou un acide organique pharmaceutiquement acceptable.

### Exemple de composition pharmaceutique

Formule de préparation pour 1000 comprimés contenant chacun 10 mg de l'ngrédient actif :

| | |
|---|---|
| Ingrédient actif | 10 g |
| Lactose | 100 g |
| Amidon de blé | 10 g |
| Stéarate de magnésium | 3 g |
| Talc | 3 g |

## Revendications

1. Composés de formule générale (1) : 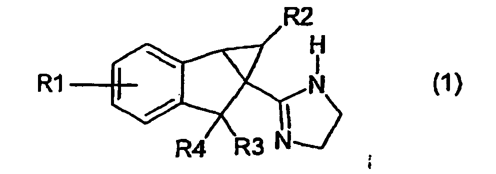 dans laquelle :
- R1 représente un atome d'hydrogène, un atome de fluor ou un groupe méthoxyle (OCH₃). Le substituant R1 sur le carbocycle aromatique peut occuper la position 2, 3, 4 ou 5 ;
- R2 représente un atome d'hydrogène ou un groupe méthyle ;
- R3 représente un atome d'hydrogène, un groupe méthyle, un groupe hydroxyle (OH) ou un groupe méthoxyle (OCH₃) ;
- R4 est un atome d'hydrogène ou
- R3 et R4 représentent ensemble un groupe carbonyle (C=O) ;
leurs sels d'addition et éventuellement les hydrates des sels d'addition avec les acides minéraux ou les acides organiques pharmaceutiquement acceptables ainsi que leurs isomères et leurs tautomères.

2. Dérivés selon la revendication 1, **caractérisé en ce qu'**ils sont choisis parmi les composés suivants :
2-(1a,6-Dihydro-1H-cyclopropa[a]indén-6a-yl)-4,5-dihydro-1H-imidazole ;
2-(1-Méthyl-1a,6-dihydro-1H-cyclopropa[a]indén-6a-yl)-4,5-dihydro-1H-imidazole ;
2-(2-Fluoro-1-méthyl-1a,6-dihydro-1H-cyclopropa[a]indén-6a-yl)-4,5-dihydro-1H-imidazole ;
2-(3-Fluoro-1-méthyl-1a,6-dihydro-1H-cyclopropa[a]indén-6a-yl)-4,5-dihydro-1H-imidazole ;
2-(4-Fluoro-1-méthyl-1a,6-dihydro-1H-cyclopropa[a]indén-6a-yl)-4,5-dihydro-1H-imidazole ;
2-(5-Fluoro-1-méthyl-1a,6-dihydro-1H-cyclopropa[a]indén-6a-yl)-4,5-dihydro-1H-imidazole ;
2-(6-Méthyl-1a,6-dihydro-1H-cyclopropa[a]indén-6a-yl)-4,5-dihydro-1H-imidazole ;
6a-(4,5-Dihydro-1H-imidazol-2-yl)-1a,6a-dihydro-1H-cyclopropa[a]indén-6-one ;
6a-(4,5-Dihydro-1H-imidazol-2-yl)-1,1a,6,6a-tétrahydro-cyclopropa[a]indén-6-ol ;
2-(6-Méthoxy-1a,6-dihydro-1H-cyclopropa[a]indén-6a-yl)-4,5-dihydro-1H-imidazole ;
2-(2,6-Diméthoxy-1a,6-dihydro-1H-cyclopropa[a]indén-6a-yl)-4,5-dihydro-1H-imidazole ;
2-(2-Fluoro-6-méthoxy-1a,6-dihydro-1H-cyclopropa[a]indén-6a-yl)-4,5-dihydro-1H-imidazole ;
2-(3-Fluoro-6-méthoxy-1a,6-dihydro-1H-cyclopropa[a]indén-6a-yl)-4,5 dihydra-1H-imidazole ;
2-(4-Fluoro-6-méthoxy-1a,6-dihydro-1H-cyclopropa[a]indén-6a-yl)-4,5-dihydro-1H-imidazole ;
2-(5-Fluoro-6-méthoxy-1a,6-dihydro-1H-cyclopropa[a]indén-6a-yl)-4,5-dihydro-1H-imidazole ;
leurs sels d'addition et éventuellement les hydrates des sels d'addition avec les acides minéraux où les acides organiques pharmaceutiquement acceptables ainsi que leurs isomères et leurs tautomères.

3. Un procédé de préparation des composés de formule (1) selon les revendications 1 et 2, **caractérisé en ce que** l'on utilise comme intermédiaire de synthèse un composé de formule (VII) : 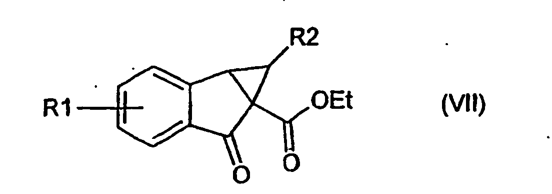 dans laquelle R1 et R2 ont la même signification que dans la formule (1) que l'on fait réagir :
- soit avec du bromure de méthyltriphénylphosphonium en présence d'une base et que l'on réduit.ensuite le composé obtenu par le diimide puis que l'on traite le produit formé par l'éthylène diamine pour obtenir le composé de formule (X), cas particulier des composé de formule (1) 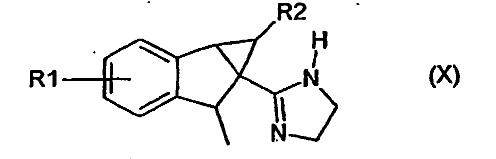 dans laquelle R1 a la même signification que dans la formule (1) et R2 est un atome d'hydrogène ou un groupe méthyle, leurs sels d'addition et éventuellement les hydrates des sels d'addition avec les acides minéraux ou les acides organiques pharmaceutiquement acceptables ainsi que leurs isomères et leurs tautomères.
- soit avec un agent réducteur et que l'on traite le composé formé par l'éthylène diamine pour obtenir le composé de formule (XII), cas particulier des composé de formule (1) 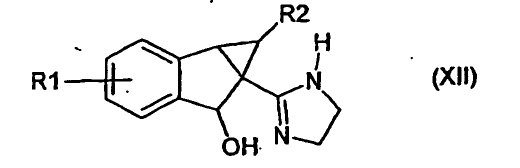 dans laquelle R1 et R2 ont la même signification que dans la formule (1), leurs sels d'addition et éventuellement les hydrates des sels d'addition avec les acides minéraux ou les acides organiques pharmaceutiquement acceptables ainsi que leurs isomères et leurs tautomères. L'oxydabon du composé de formule (XII) conduit au composé de formule (XIII), cas particulier des composé de formule (1) 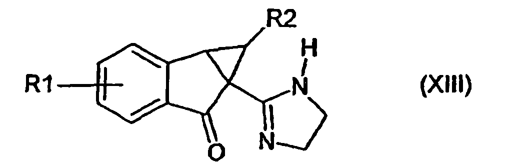 dans laquelle R1 et R2 ont la même signification que dans la formule (1), leurs sels d'addition et éventuellement les hydrates des sels d'addition avec les acides minéraux ou les acides organiques pharmaceutiquement acceptables ainsi que leurs isomères et leurs tautomères.
- soit avec un agent réducteur et que l'on méthyle le composé obtenu en présence d'iodure de méthyle et de sel d'argent puis que l'on traite le produit formé par l'éthylène diamine pour obtenir le composé de formule (XV), cas particulier des composé de formule (1) 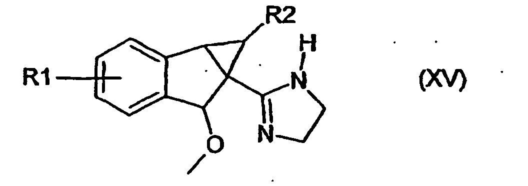 dans laquelle R1 et R2 ont la même signification que dans la formule (1), leurs sels d'addition et éventuellement les hydrates des sels d'addition avec les acides minéraux ou les acides organiques pharmaceutiquement acceptables ainsi que leurs isomères et leurs tautomères.
- soit avec un agent réducteur en milieu acide fort et que l'on traite le composé formé par l'éthylène diamine pour obtenir le composé de formule (XVII), cas particulier des composé de formule (1) 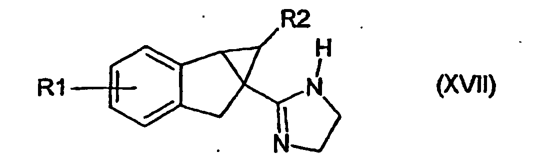
dans laquelle R1 et R2 ont la même signification que dans la formule (1), leurs sels d'addition et éventuellement les hydrates des sels d'addition avec les acides minéraux ou les acides organiques pharmaceutiquement acceptables ainsi que leurs isomères et leurs tautomères.

4. Dérivés selon la revendication 1, **caractérisé en ce que** les substituants R2 et 4,5-dihydro-1H-imidazole occupent des positions syn-périplanaires par rapport au plan défini par le noyau cyclopropanique, R1, R3 et R4 ont la même signification que dans la formule (1) et R2 est un groupe méthyle (CH₃).

5. Dérivés selon la revendication 1; **caractérisé en ce que** les substituants R3 et 4,5-dihydro-1H-imidazole occupent des positions anti-périplanaires par rapport au plan défini par le noyau indanique, R1 et R2 ont la même signification que dans la formule (1), R3 représente un groupe méthyle, un groupe hydroxyle (OH) ou un groupe méthoxyle (OCH₃) et R4 est un atome d'hydrogène.

6. Dérivés de formule générale (1), selon l'une quelconque des revendications 1, 2, 4 ou 5 **caractérisé en ce qu'**il est choisi parmi l'énantiomère lévogyre ou l'énantiomère dextrogyre des composés de formule générale (1).

7. Composé selon l'une des revendication 1, 2, 4, 5 et 6 à titre de médicaments.

8. Compositions pharmaceutiques **caractérisées en ce qu'**elles contiennent comme ingrédient actif au moins un composé selon l'une des revendications 1, 2, 4, 5 et 6 associé à un support pharmaceutique inerte ou autres véhicules pharmaceutiquement acceptables et éventuellement à un autre médicament

9. Utilisation d'un composé selon l'une des revendications 1, 2 et 4 à 6 pour la fabrication d'un médicament utile dans le traitement de la maladie d'Alzheimer ou utile dans le traitement de l'évolution de la maladie d'Alzheimer.

10. Utilisation d'un composé selon l'une des revendications 1, 2 et 4 à 6 pour la fabrication d'un médicament utile dans le traitement ou dans le traitement de l'évolution de la maladie de Parkinson ou de la maladie de Creutzfeld Jacob ou de l'accident vasculaire cérébral.

## Patentansprüche

1. Verbindungen der allgemeinen Formel (1): 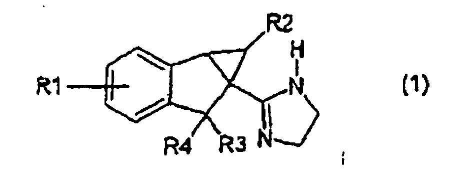 in welcher:
- R1 für ein Wasserstoffatom, ein Fluoratom oder eine Methoxygruppe (OCH₃) steht. Der Substituent R1 an dem aromatischen Carbocyclus kann die Position 2, 3, 4 oder 5 einnehmen;
- R2 für ein Wasserstoffatom oder eine Methylgruppe steht;
- R3 für ein Wasserstoffatom, eine Methylgruppe, eine Hydroxylgruppe (OH) oder eine Methoxygruppe (OCH₃) steht;
- R4 ein Wasserstoffatom ist oder
- R3 und R4 zusammen für eine Carbonylgruppe (C=O) stehen;
deren Additionssalze und gegebenenfalls die Hydrate der Additionssalze mit den anorganischen Säuren oder den organischen Säuren, die pharmazeutisch annehmbar sind, wie auch deren Isomere und deren Tautomere.

2. Derivate nach Anspruch 1, **dadurch gekennzeichnet, dass** sie unter den folgenden Verbindungen ausgewählt werden:
2-(1 a,6-Dihydro-1H-cyclopropa[a]inden-6a-yl)-4,5-dihydro-1H-imidazol;
2-(1-Methyl-1 a,6-dihydro-1H-cyclopropa[a]inden-6a-yl)-4,5-dihydro-1H-imidazol;
2-(2-Fluor-1-methyl-1a,6-dihydro-1H-cyclopropa[a]inden-6a-yl)-4,5-dihydro-1H-imidazol;
2-(3-Fluor-1-methyl-1a,6-dihydro-1H-cyclopropa[a]inden-6a-yl)-4,5-dihydro-1H-imidazol;
2-(4-Fluor-1-methyl-1 a,6-dihydro-1H-cyclopropa[a]inden-6a-yl)-4,5-dihydro-1H-imidazol;
2-(5-Fluor-1-methyl-1 a,6-dihydro-1H-cyclopropa[a]inden-6a-yl)-4,5-dihydro-1H-imidazol;
2-(6-Methyl-1 a,6-dihydro-1H-cyclopropa[a]inden-6a-yl)-4,5-dihydro-1H-imidazol;
6a-(4,5-Dihydro-1H-imidazol-2-yl)-1 a,6a-dihydro-1H-cyclopropa[a]inden-6-on;
6a-(4,5-Dihydro-1H-imidazol-2-yl)-1,1a,6,6a-tetrahydro-cyclopropa[a]inden-6-ol;
2-(6-Methoxy-1a,6-dihydro-1H-cyclopropa[a]inden-6a-yl)-4,5-dihydro-1H-imidazol;
2-(2,6-Dimethoxy-1 a,6-dihydro-1H-cyclopropa[a]inden-6a-yl)-4,5-dihydro-1H-imidazol;
2-(2-Fluor-6-methoxy-1 a,6-dihydro-1H-cyclopropa[a]inden-6a-yl)-4,5-dihydro-1H-imidazol;
2-(3-Fluor-6-methoxy-1a,6-dihydro-1H-cyclopropa[a]inden-6a-yl)-4,5-dihydro-1H-imidazol;
2-(4-Fluor-6-methoxy-1a,6-dihydro-1H-cyclopropa[a]inden-6a-yl)-4,5-dihydro-1H-imidazol;
2-(5-Fluor-6-methoxy-1a,6-dihydro-1H-cyclopropa[a]inden-6a-yl)-4,5-dihydro-1H-imidazol;
deren Additionssalze und gegebenenfalls die Hydrate der Additionssalze mit den anorganischen Säuren oder den organischen Säuren, die pharmazeutisch annehmbar sind, sowie deren Isomere und deren Tautomere.

3. Verfahren zur Herstellung der Verbindungen der Formel (1) nach den Ansprüchen 1 und 2, **dadurch gekennzeichnet, dass** man als Synthese-Zwischenprodukt eine Verbindung der Formel (VII) einsetzt: 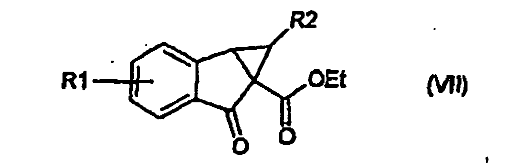 in welcher R1 und R2 die gleiche Bedeutung wie in der Formel (1) haben, dass man diese reagieren lässt:
- entweder mit Methyltriphenylphosphoniumbromid in Gegenwart einer Base und dass man dann die erhaltene Verbindung durch Diimid reduziert, dass man dann das gebildete Produkt mit Ethylendiamin behandelt, um die Verbindung der Formel (X) zu erhalten, ein besonderer Fall der Verbindung der Formel (1) 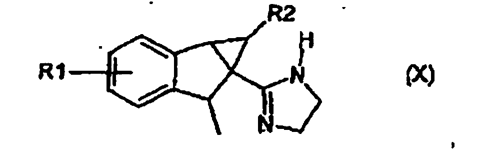 in welcher R1 die gleiche Bedeutung wie in der Formel (1) hat und R2 ein Wasserstoffatom oder eine Methylgruppe ist, deren Additionssalze und gegebenenfalls die Hydrate der Additionssalze mit den anorganischen Säuren oder den organischen Säuren, die pharmazeutisch annehmbar sind, sowie deren Isomere und deren Tautomere.
- oder mit einem Reduktionsmittel und dass man die gebildete Verbindung mit Ethylendiamin behandelt, um die Verbindung der Formel (XII) zu erhalten, ein besonderer Fall der Verbindung der Formel (1) 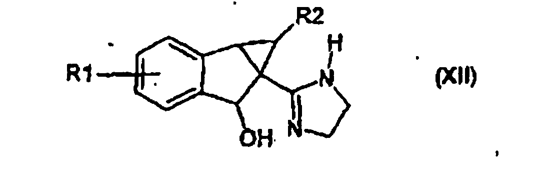 in welcher R1 und R2 die gleiche Bedeutung wie in der Formel (1) haben, deren Additionssalze und gegebenenfalls die Hydrate der Additionssalze mit den anorganischen Säuren oder den organischen Säuren, die pharmazeutisch annehmbar sind, sowie deren Isomere und deren Tautomere. Die Oxidation der Verbindung der Formel (XII) führt zu der Verbindung der Formel (XIII), ein besonderer Fall der Verbindung der Formel (1) 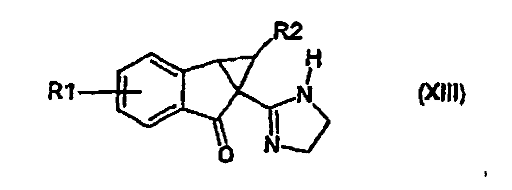 in welcher R1 und R2 die gleiche Bedeutung wie in der Formel (1) haben, deren Additionssalze und gegebenenfalls die Hydrate der Additionssalze mit den anorganischen Säuren oder den organischen Säuren, die pharmazeutisch annehmbar sind, sowie deren Isomere und deren Tautomere.
- oder mit einem Reduktionsmittel und dass man die erhaltene Verbindung in Gegenwart von Methyliodid und eines Silbersalzes methyliert, dass man dann das gebildete Produkt mit Ethylendiamin behandelt, um die Verbindung der Formel (XV) zu erhalten, ein besonderer Fall der Verbindung der Formel (1), 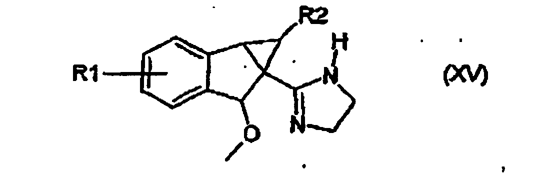 in welcher R1 und R2 die gleiche Bedeutung wie in der Formel (1) haben, deren Additionssalze und gegebenenfalls die Hydrate der Additionssalze mit den anorganischen Säuren oder den organischen Säuren, die pharmazeutisch annehmbar sind, sowie deren Isomere und deren Tautomere.
- oder mit einem Reduktionsmittel in einem Milieu einer starken Säure und dass man die gebildete Verbindung mit Ethylendiamin behandelt, um die Verbindung der Formel (XVII) zu erhalten, ein besonderer Fall der Verbindung der Formel (1), 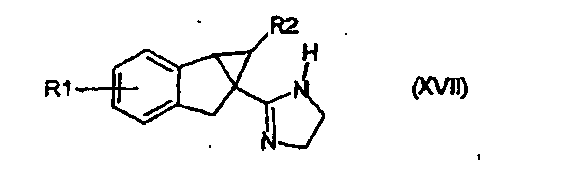
in welcher R1 und R2 die gleiche Bedeutung wie in der Formel (1) haben, deren Additionssalze und gegebenenfalls die Hydrate der Additionssalze mit den anorganischen Säuren oder den organischen Säuren, die pharmazeutisch annehmbar sind, sowie deren Isomere und deren Tautomere.

4. Derivate nach Anspruch 1, **dadurch gekennzeichnet, dass** die Substituenten R2 und 4,5-Dihydro-1H-imidazol synperiplanare Positionen bezogen auf die durch den Cyclopropanring definierte Ebene einnehmen, R1, R3 und R4 die gleiche Bedeutung wie in der Formel (1) haben und R2 eine Methylgruppe (CH₃) ist.

5. Derivate nach Anspruch 1, **dadurch gekennzeichnet, dass** die Substituenten R3 und 4,5-Dihydro-1H-imidazol antiperiplanare Positionen bezogen auf die durch den Indanring definierte Ebene einnehmen, R1 und R2 die gleiche Bedeutung wie in der Formel (1) haben, R3 für eine Methylgruppe, eine Hydroxylgruppe (OH) oder eine Methoxygruppe (OCH₃) steht und R4 ein Wasserstoffatom ist.

6. Derivate der allgemeinen Formel (1) nach einem der Ansprüche 1, 2, 4 oder 5, **dadurch gekennzeichnet, dass** es unter dem linksdrehenden Enantiomer oder dem rechtsdrehenden Enantiomer der Verbindungen der allgemeinen Formel (1) ausgewählt wird.

7. Verbindung nach einem der Ansprüche 1, 2, 4, 5 und 6 als Arzneimittel.

8. Pharmazeutische Zusammensetzungen, **dadurch gekennzeichnet, dass** sie als Wirkstoff wenigstens eine Verbindung nach einem der Ansprüche 1, 2, 4, 5 und 6 kombiniert mit einem inerten pharmazeutischen Träger oder anderen, pharmazeutisch annehmbaren Vehikeln und gegebenenfalls mit einem anderen Arzneimittel enthalten.

9. Verwendung einer Verbindung nach einem der Ansprüche 1, 2 und 4 bis 6 für die Herstellung eines Arzneimittels, welches bei der Behandlung der Alzheimer'-Krankheit nützlich ist oder bei der Behandlung des Verlaufs der Alzheimer'-Krankheit nützlich ist.

10. Verwendung einer Verbindung nach einem der Ansprüche 1, 2 und 4 bis 6 für die Herstellung eines Arzneimittels, welches bei der Behandlung oder bei der Behandlung des Verlaufs der Parkinson'-Krankheit oder der Creutzfeld-Jacob-Krankheit oder des Schlaganfalls nützlich ist.

## Claims

1. Compounds of general formula (1): 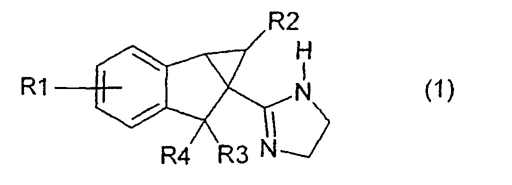 in which:
- R1 represents a hydrogen atom, a fluorine atom or a methoxy (OCH₃) group. The substituent R1 on the aromatic carbocycle may be in position 2, 3, 4 or 5;
- R2 represents a hydrogen atom or a methyl group;
- R3 represents a hydrogen atom, a methyl group, a hydroxyl group (OH) or a methoxy group (OCH₃);
- R4 is a hydrogen atom or
- R3 and R4 together represent a carbonyl group (C=O) ;
the addition salts thereof and optionally the hydrates of the addition salts with pharmaceutically acceptable mineral acids or organic acids, and also the tautomeric and isomeric forms thereof.

2. Derivatives according to Claim 1, **characterized in that** they are chosen from the following compounds:
2-(1a,6-dihydro-1H-cyclopropa[a]inden-6a-yl)-4,5-dihydro-1H-imidazole;
2-(1-methyl-1a,6-dihydro-1H-cyclopropa[a]inden-6a-yl)-4,5-dihydro-1H-imidazole;
2-(2-fluoro-1-methyl-1a,6-dihydro-1H-cyclopropa[a]-inden-6a-yl)-4,5-dihydro-1H-imidazole;
2-(3-fluoro-1-methyl-1a,6-dihydro-1H-cyclopropa[a]-inden-6a-yl)-4,5-dihydro-1H-imidazole;
2-(4-fluoro-1-methyl-1a,6-dihydro-1H-cyclopropa[a]-inden-6a-yl)-4,5-dihydro-1H-imidazole;
2-(5-fluoro-1-methyl-1a,6-dihydro-1H-cyclopropa[a]-inden-6a-yl)-4,5-dihydro-1H-imidazole;
2-(6-methyl-1a,6-dihydro-1H-cyclopropa[a]inden-6a-yl)-4,5-dihydro-1H-imidazole;
6a-(4,5-dihydro-1H-imidazol-2-yl)-1a,6a-dihydro-1H-cyclopropa[a]inden-6-one;
6a-(4,5-dihydro-1H-imidazol-2-yl)-1,1a,6,6a-tetrahydro-cyclopropa[a]inden-6-ol;
2-(6-methoxy-1a,6-dihydro-1H-cyclopropa[a]inden-6a-yl)-4,5-dihydro-1H-imidazole;
2-(2,6-dimethoxy-1a,6-dihydro-1H-cyclopropa[a]inden-6a-yl)-4,5-dihydro-1H-imidazole;
2-(2-fluoro-6-methoxy-1a,6-dihydro-1H-cyclopropa[a]-inden-6a-yl)-4,5-dihydro-1H-imidazole;
2-(3-fluoro-6-methoxy-1a,6-dihydro-1H-cyclopropa[a]-inden-6a-yl)-4,5-dihydro-1H-imidazole;
2-(4-fluoro-6-methoxy-1a,6-dihydro-1H-cyclopropa[a]-inden-6a-yl)-4,5-dihydro-1H-imidazole;
2-(5-fluoro-6-methoxy-1a,6-dihydro-1H-cyclopropa[a]-inden-6a-yl)-4,5-dihydro-1H-imidazole;
the addition salts thereof and optionally the hydrates of the addition salts with pharmaceutically acceptable mineral acids or organic acids, and also the tautomeric and isomeric forms thereof.

3. A process for preparing compounds of formula (1) according to Claims 1 and 2, **characterized in that** a synthetic intermediate used is a compound of formula (VII): 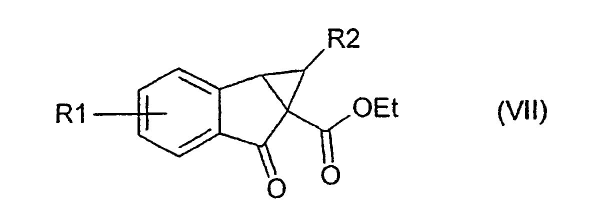 in which R1 and R2 have the same meaning as in formula (1), which is reacted:
- either with methyltriphenylphosphonium bromide in the presence of a base, and the compound obtained is then reduced with diimide and the product formed is then treated with ethylenediamine to obtain the compound of formula (X), a particular case of the compounds of formula (1) 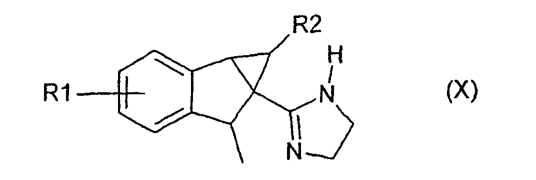 in which R1 has the same meaning as in formula (1) and R2 is a hydrogen atom or a methyl group, the addition salts thereof and optionally the hydrates of the addition salts with pharmaceutically acceptable mineral acids or organic acids, and also the tautomeric and isomeric forms thereof,
- or with a reducing agent, and the compound formed is treated with ethylenediamine to obtain the compound of formula (XII), a particular case of the compounds of formula (1) 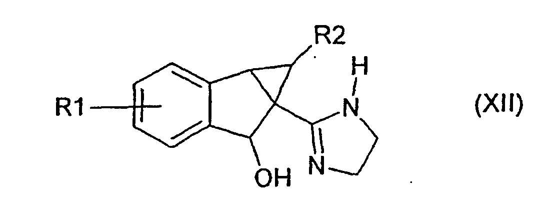 in which R1 and R2 have the same meaning as in formula (1), the addition salts thereof and optionally the hydrates of the addition salts with pharmaceutically acceptable mineral acids or organic acids, and also the tautomeric and isomeric forms thereof. Oxidation of the compound of formula (XII) leads to the compound of formula (XIII), a particular case of the compounds of formula (1) 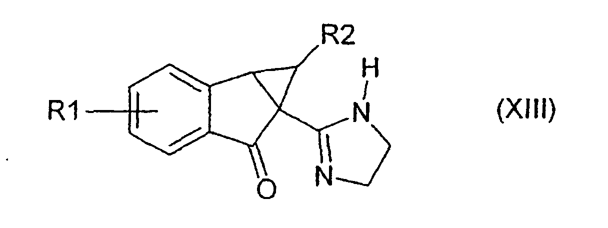 in which R1 and R2 have the same meaning as in formula (1), the addition salts thereof and optionally the hydrates of the addition salts with pharmaceutically acceptable mineral acids or organic acids, and also the tautomeric and isomeric forms thereof,
- or with a reducing agent, and the compound obtained is methylated in the presence of methyl iodide and a silver salt, and the product formed is then treated with ethylenediamine to obtain the compound of formula (XV), a particular case of the compounds of formula (1) 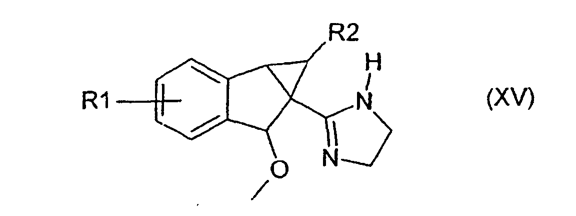 in which R1 and R2 have the same meaning as in formula (1), the addition salts thereof and optionally the hydrates of the addition salts with pharmaceutically acceptable mineral acids or organic acids, and also the tautomeric and isomeric forms thereof,
- or with a reducing agent in strong acid medium, and the compound formed is treated with ethylenediamine to obtain the compound of formula (XVII), a particular case of the compounds of formula (1) 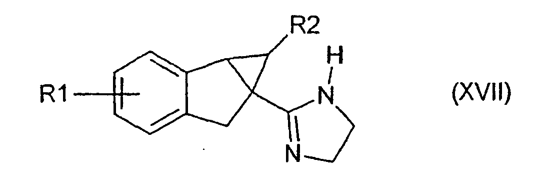
in which R1 and R2 have the same meaning as in formula (1), the addition salts thereof and optionally the hydrates of the addition salts with pharmaceutically acceptable mineral acids or organic acids, and also the tautomeric and isomeric forms thereof.

4. Derivatives according to Claim 1, **characterized in that** the substituents R2 and 4,5-dihydro-1H-imidazole are in syn-periplanar positions relative to the plane defined by the cyclopropane ring, R1, R3 and R4 have the same meaning as in formula (1) and R2 is a methyl group (CH₃).

5. Derivatives according to Claim 1, **characterized in that** the substituents R3 and 4,5-dihydro-1H-imidazole are in anti-periplanar positions relative to the plane defined by the indane ring system, R1 and R2 have the same meaning as in formula (1), R3 represents a methyl group, a hydroxyl group (OH) or a methoxy group (OCH₃) and R4 is a hydrogen atom.

6. Derivatives of general formula (1) according to any one of Claims 1, 2, 4 and 5, **characterized in that** they are chosen from the levorotatory enantiomer or the dextrorotarory enantiomer of the compounds of general formula (1).

7. Compound according to one of Claims 1, 2, 4, 5, and 6, as medicinal products.

8. Pharmaceutical compositions, **characterized in that** they contain, as active ingredient, at least one compound according to one of Claims 1, 2, 4, 5 and 6, combined with an inert pharmaceutical support or other pharmaceutically acceptable vehicles and optionally with another medicinal product.

9. Use of a compound according to one of Claims 1, 2 and 4 to 6, for the manufacture of a medicinal product that is useful in the treatment of Alzheimer's disease or useful in the treatment of the evolution of Alzheimer's disease.

10. Use of a compound according to one of Claims 1, 2 and 4 to 6 for the manufacture of a medicinal product that is useful in the treatment or in the treatment of the evolution of Parkinson's disease or Creutzfeld-Jacob disease or strokes.
